# EUROPÄISCHE PATENTSCHRIFT

(11) **EP 1 844 744 B1**
(45) Veröffentlichungstag und Bekanntmachung des Hinweises auf die Patenterteilung: **27.07.2011**
(21) Anmeldenummer: 06007601.5
(22) Anmeldetag: 11.04.2006
(51) Int. Cl.: A61F 9/009

(54) **Lasereinrichtung für die ophthalmologische Chirurgie**
Laser arrangement for ophthalmic surgery
Dispositif laser pour la chirurgie ophtalmique

(43) Veröffentlichungstag der Anmeldung: 17.10.2007
(60) Teilanmeldung: 10008851.7
(73) Patentinhaber: WaveLight GmbH, 91058 Erlangen (DE)
(72) Erfinder: Wüllner, Christian, 91096 Möhrendorf (DE); Mrochen, Michael, Dr., 8606 Werrikon (CH); Donitzky, Christof, 90542 Eschenau (DE)
(74) Vertreter: Katérle, Axel

(56) Entgegenhaltungen:
- EP-A- 1 537 841
- WO-A-01/95842
- WO-A-94/09849
- US-A- 5 347 326
- US-B1- 6 325 792

## Beschreibung

Die Erfindung betrifft eine Lasereinrichtung für die ophthalmologische Chirurgie, mit einer gepulste Laserstrahlung bereitstellenden Laserstrahlungsquelle und Mitteln zur Einkopplung der Laserstrahlung in einen okularen Behandlungsort, wobei die Einkopplungsmittel eine auf eine Augenoberfläche aufzusetzende Applanationslinse umfassen.

Gepulste Laserstrahlung wird in der Augenchirurgie beispielsweise zur Anbringung von Schnitten in der Hornhaut (Kornea) oder zum Abtrag (Ablation) von Material von der Hornhautoberfläche verwendet. Die eingestrahlte Laserstrahlung bewirkt im Hornhautgewebe einen photodisruptiven Prozess, der zur Gewebetrennung bzw. zur Verdampfung von Gewebematerial führt. Solche Bearbeitungen der Kornea finden beispielsweise im Rahmen von refraktiven Verfahren zur Minderung oder vollständigen Behebung von Fehlsichtigkeiten des Auges statt, bei denen die Kornea neugeformt wird und hierdurch ihre Brechungseigenschaften verändert werden.

Eines von mehreren gängigen refraktiven Verfahren der Hornhautchirurgie ist die sogenannte LASIK (Laser in-situ Keratomileusis). Hierbei wird aus dem Hornhautepithel entweder mechanisch (mittels einer oszillierenden Schneidklinge in einem sogenannten Mikrokeratom) oder optisch (mittels Laserstrahlung) ein Deckelchen herausgeschnitten, das in einem Teil seines Rands noch an der Hornhaut hängt. Anschließend wird dieses üblicherweise als Flap bezeichnete Deckelchen zur Seite geklappt, wodurch das darunter liegende Stroma zugänglich wird. Mit Laserstrahlung wird dann nach Maßgabe eines zuvor für den jeweiligen Patienten ermittelten Ablationsprofils Stromagewebe abgetragen. Der Flap wird danach wieder zurückgeklappt, wodurch die Wunde relativ schnell verheilen kann.

Je nach Art der Bearbeitung (z.B. Inzision oder Ablation) oder/und Gewebetyp wird in der laseroptischen Augenchirurgie Laserstrahlung unterschiedlicher Wellenlängen oder/und Pulsdauern eingesetzt. Für die Anbringung von Schnitten in der Hornhaut (etwa für die Präparation eines Flaps) beispielsweise ist es üblich, Laserstrahlung im niederen infraroten (NIR) Wellenlängenbereich, beispielsweise zwischen 1000 und 1100 nm, mit Pulsdauern im Femtosekundenbereich oder im niederen Pikosekundenbereich einzusetzen. Dagegen wird für die Photoablation von Stromagewebe in der Regel auf Laserstrahlung im ultravioletten Wellenlängenbereich, beispielsweise 193 nm oder 347 nm, zurückgegriffen, wobei die verwendeten Pulsdauern auch länger sein können, bis hin in den Nanosekundenbereich.

Für eine präzise Einkopplung der Laserstrahlung in das Auge ist es bekannt, das Auge mittels einer Fixationsvorrichtung zu fixieren, welche durch Vakuum am Auge angesaugt wird. Die Fixationsvorrichtung umfasst eine als distales Einkoppelelement für die Laserstrahlung dienende Applanationslinse, die beim Ansaugen der Fixationsvorrichtung in direktem Kontakt mit der Augenoberfläche zu Sitzen kommt. Durch die Applanationslinse wird eine definierte, stabile Schnittstelle zwischen dem Auge und dem Lasersystem geschaffen. Beispiele von Fixationsvorrichtungen und Applanationslinsen finden sich in US 2002/0103481 A1, EP 0 608 052 A2, EP 0 993 814 A1 und US 5,549,632.

Bisherige Applanationslinsen erstrecken sich über weite Bereiche der Hornhaut und sitzen unbewegt auf der Hornhaut. Es gibt sie in unterschiedlichsten Formen. Durch das Ansaugen der Fixationsvorrichtung werden sie so gegen das Auge gedrückt, dass sich die Hornhaut verformt und flächig an die Linse anschmiegt. Besonders bei planparallelen Applanationslinsen und bei konvex gekrümmten Linsen wird dabei eine vergleichsweise hohe biomechanische Belastung auf die Hornhaut ausgeübt. Außerdem wird durch die Verformung des Auges der Augeninnendruck vergleichsweise stark erhöht. Es sind auch Applanationslinsen mit einer konkav geformten, rotationssymmetrischen Anlagefläche auf ihrer augenzugewandten Seite bekannt, und zwar sowohl mit sphärischer als auch mit asphärischer Krümmung. Mit beiden konkaven Linsenvarianten lassen sich zwar die biomechanische Belastung der Hornhaut und der erhöhte Augeninnendruck abschwächen. Es sind jedoch weitere Verbesserungen in dieser Hinsicht nötig.

WO 94/09849 A1 beschreibt im Zusammenhang mit seiner Fig. 7 verschiedene Formen einer für eine Laserbearbeitung der Kornea auf das Auge aufzusetzenden Linse. Durch das Aufsetzen der Linse soll eine Verformung der Kornea eintreten. Speziell wird eine Linsenform vorgeschlagen, bei der die Kornea an ihrem Rand stärker eingedrückt wird als in ihrer Mitte. Dies soll zu einer näherungsweise flachen Komeaoberfläche führen, die dann mittels eines Laserstrahls lediglich zweidimensional abgefahren zu werden braucht. Generell werden sphärische, asphärische oder torische Unsen angesprochen.

Aufgabe der Erfindung ist es, eine Linsengeometrie anzugeben, die eine weitere Reduzierung des Augeninnendrucks und der Hornhautbelastung gestattet.

Zur Lösung dieser Aufgabe sieht die Erfindung eine Lasereinrichtung nach Anspruch 1 vol.

Die Erfindung macht sich die Erkenntnis zunutze, dass die Hornhautoberfläche des menschlichen Auges nicht rotationssymmetrisch ist, sondern entlang unterschiedlicher Meridiane unterschiedlichen Krümmungsverlauf besitzt. Insbesondere kann die Hornhautoberfläche in guter Näherung und mit guter Allgemeingültigkeit durch eine bitorische (bikomische) Fläche modelliert werden, welche in zwei zueinander senkrechten Meridianschnitten unterschiedliche Krümmungsradien besitzt und in beiden Meridianrichtungen asphärisch ist. In dem Artikel "Custom photorefractive keratectomy of sperical and cylindrical refractive error and higher-order aberration" von Jim Schwiegerling und Robert W. Snyder, erschienen in J. Opt. Soc. Am. A, Bd. 15, Nr. 9, September 1998, Seiten 2572-2579, ist eine mathematische Formel (dort Gleichung (6)) zur Beschreibung einer bitorischen Fläche in Polarkoordinaten angegeben. Die Kontaktfläche der erfindungsgemäßen Applanationslinse kann beispielsweise dieser Formel nachgebildet sein. Es versteht sich, dass die Kontaktfläche der Applanationslinse nicht im streng mathematischen Sinn exakt bitorisch sein muss, solange sie in zwei quer (nicht notwendigerweise exakt senkrecht) zueinander verlaufenden Meridianrichtungen jeweils asphärische Kontur mit unterschiedlichen Krümmungsradien hat. Die Kontaktfläche der Applanationslinse kann beispielsweise auf der Basis von Daten gebildet sein, welche durch Vermessung der Hornhautoberfläche einer oder mehrerer Personen gewonnen wurden. So kann beispielsweise die Applanationslinse für jeden Patienten individuell angefertigt werden oder es können die Daten einer Vielzahl von Personen in eine oder mehrere Standardlinsen umgesetzt werden.

Aufgrund der besseren Anpassung der Kontaktfläche der Linse an die tatsächliche Form der Hornhautoberfläche muss sich die Hornhaut weniger verformen, um sich perfekt an die Kontaktfläche anzuschmiegen. Deshalb ist ihre biomechanische Belastung geringer und auch der Augeninnendruck steigt nicht so stark an.

Die Erfindung stellt ferner gemäß Anspruch 2 eine Applanationslinse zur Verwendung in einer Lasereinrichtung der vorstehend beschriebenen Art bereit. Die Applanationslinse besitzt hierbei auf einer zur Anlage an einer Augenoberfläche bestimmten Linsenseite eine zumindest näherungsweise bitorische Kontaktfläche. Basierend auf empirischen Daten, die durch Vermessung der Hornhautoberfläche einer Vielzahl von Personen gewonnen werden können, ist es möglich, einen Satz von Applanationslinsen anzufertigen, die sich durch unterschiedliche Krümmungsradien oder/und unterschiedliche Asphärizitäten ihrer Kontaktfläche unterscheiden.

Die Erfindung wird nachfolgend anhand der beigefügten Zeichnungen weiter erläutert. Es stellen dar:
Figur 1 in starker schematischer Vereinfachung eine Lasereinrichtung für die ophthalmologische Chirurgie,
Figur 2 schematisch die menschliche Hornhautoberfläche,
Figur 3 im Schnitt und schematisch eine auf ein Auge aufgesetzte bitorische Applanationslinse gemäß einem Ausführungsbeispiel,
Figur 4 eine Schnittansicht der Applanationslinse der Figur 3 entlang der dortigen Schnittrichtung IV-IV,
Figur 5 im Schnitt schematisch eine auf ein Auge aufgesetzte Stablinse gemäß einem Ausführungsbeispiel,
Figur 6 in schematischer Draufsicht eine linear über eine Hornhautoberfläche bewegte Stablinse und
Figur 7 in schematischer Draufsicht eine durch Drehung über eine Hornhautoberfläche bewegbare Stablinse.

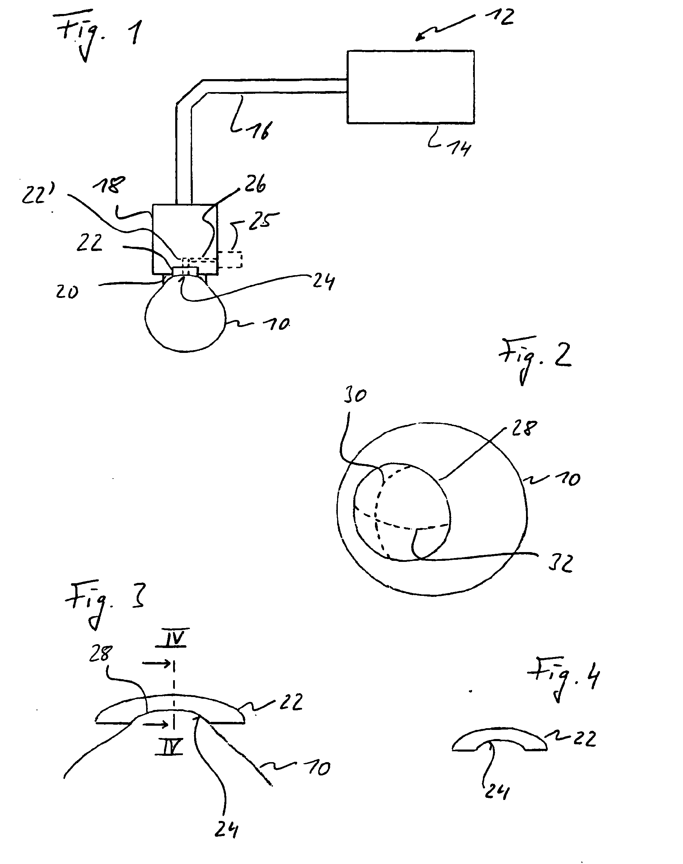

Es wird zunächst auf Figur 1 verwiesen. Dort ist schematisch ein menschliches Auge 10 angedeutet, dessen Hornhaut mit einer Lasereinrichtung 12 bearbeitet wird, beispielsweise zur Erzeugung eines Flap-Schnitts im Rahmen einer LASIK-Behandlung. Die Lasereinrichtung 12 umfasst eine Laserstrahlungsquelle 14, welche gepulste Laserstrahlung im NIR- oder UV-Wellenlängenbereich erzeugt. Vorzugsweise liegt die Pulsdauer der erzeugten Laserstrahlung im Bereich von Femtosekunden, sie kann aber auch im Piko- oder sogar im Nanosekundenbereich liegen. Die Laserstrahlungsquelle 14 kann beispielsweise einen Faserlaser, einen Festkörperlaser oder einen Excimer-Laser enthalten. Beschränkungen hinsichtlich des Typs der Laserstrahlungsquelle 14, der Wellenlänge der von ihr erzeugten Laserstrahlung und der Pulslängen sind im Rahmen der Erfindung nicht beabsichtigt.

Die von der Laserstrahlungsquelle 14 bereitgestellten Strahlungspulse werden über eine Strahlführungsanordnung 16 zu einer Einkoppeleinheit 18 geleitet, mittels welcher die Pulse in die Hornhaut des Auges 10 eingekoppelt werden. Die Einkoppeleinheit 18 umfasst Fixationsmittel, mittels welcher die Einkoppeleinheit 18 oder zumindest ein augennaher Teil derselben an dem Auge 10 durch Vakuumansaugung fixierbar ist. Hierzu weisen die Fixationsmittel eine vorzugsweise ringförmige Ansaugkammer 20 auf, welche bei Aufsetzen der Einkoppeleinheit 18 auf das Auge 10 durch die Augenoberfläche geschlossen wird und in nicht näher dargestellter Weise an eine Vakuumpumpe angeschlossen ist. Die Fixation des Auges durch Ansaugen einer Fixationskomponente ist in der Fachwelt an sich bekannt, weswegen auf Details der Fixationsmittel der Einkoppeleinheit 18 hier nicht näher eingegangen werden muss.

Die Strahlführungsanordnung 16 oder/und die Einkoppeleinheit 18 enthalten ferner nicht näher dargestellte Ablenkmittel (üblicherweise als Scanner bezeichnet), um die Laserstrahlung über ein zu bearbeitendes Zielgebiet hinwegbewegen zu können, sowie Fokussiermittel zur Fokussierung der in das Auge 10 eingekoppelten Strahlung auf einen Zielpunkt (Fokus). Solche Ablenk- und Fokussiermittel sind in der Fachwelt hinlänglich bekannt. Auf eine genauere Beschreibung dieser Komponenten kann deshalb an dieser Stelle verzichtet werden.

Als distales Einkoppelelement enthält die Einkoppeleinheit 18 eine Applanationslinse 22, welche im Zuge der Fixierung der Einkoppeleinheit 18 am Auge 10 zur Auflage auf der Hornhautoberfläche gelangt. Die Applanationslinse besitzt auf ihrer augenzugewandten Seite eine in Figur 1 mit 24 bezeichnete Kontaktfläche, welche bei einer Ausführungsform in näherungsweiser Anpassung an die tatsächliche Kontur der menschlichen Hornhautoberfläche im wesentlichen bitorisch ausgebildet ist. Bei dieser Ausführungsform erstreckt sich die Applanationslinse 22 über den gesamten zu bearbeitenden Bereich der Hornhaut; sie sitzt während der Operation unbewegt auf der Hornhaut. Bei einer anderen Ausführungsform, die in Figur 1 gestrichelt eingezeichnet ist, ist die Applanationslinse eine Stablinse, deren Kontaktfläche bei Betrachtung in einem Schnitt quer zur Stablängsrichtung rundlich konvex ist. Insbesondere kann die Stablinse eine Zylinderlinse sein, welche in Richtung ihrer Längserstreckung im wesentlichen geradlinig verläuft. In Figur 1 ist die Stablinse bei 22' angedeutet. Aufgrund ihres vergleichsweise kleinflächigen Kontaktbereichs mit der Hornhautoberfläche des Auges 10 ist sie bewegbar in der Einkoppeleinheit 18 angeordnet, wobei zum Antrieb der Stablinse 22' eine motorische, beispielsweise elektromotorische oder piezomotorische, Antriebseinheit 25 vorgesehen ist, welche über eine geeignete Antriebsverbindung (bei 26 angedeutet) antriebsmäßig mit der Stablinse 22' gekoppelt oder koppelbar ist.

Figur 2 zeigt das Auge 10 in einer vergrößerten Darstellung von schräg vorne. Die Hornhaut ist bei 28 angedeutet. Ihre Oberfläche hat im Regelfall keine exakte Kugelkontur, sondern eine angenähert bitorische Form. Bitorisch bedeutet, dass die Hornhautoberfläche entlang zweier quer zueinander verlaufender Hauptmeridiane jeweils asphärisch mit jeweils unterschiedlichem Krümmungsradius verläuft. Die beiden Meridiane sind in Figur 2 gestrichelt eingezeichnet und mit 30, 32 bezeichnet. Beim menschlichen Auge ist der Winkel zwischen den beiden Meridianen 30, 32 oftmals nicht exakt 90 Grad. Dennoch hat sich gezeigt, dass sich die menschliche Hornhautoberfläche mathematisch durch eine Bitorus-Fläche mit senkrecht aufeinanderstehenden Meridianen gut modellieren lässt. Dementsprechend liegen bei der bitorischen Applanationslinse 22 die Meridiane der Kontaktfläche 24 vorzugsweise senkrecht zueinander. Es ist freilich nicht ausgeschlossen, zur noch besseren Anpassung an die tatsächlichen Gegebenheiten der menschlichen Hornhautoberfläche, beispielsweise abhängig vom jeweiligen Patienten, die Meridiane der bitorischen Kontaktfläche 24 nicht exakt senkrecht aufeinanderzulegen, sondern beispielsweise in einem Winkel von 85 Grad oder einem anderen von 90 Grad verschiedenen Winkel.

Figur 3 zeigt die Applanationslinse 22 schematisch in einem Zustand, in dem sie auf die Hornhaut 28 des Auges 10 aufgesetzt ist. Die Schnittdarstellung der Figur 3 zeigt den Verlauf der Kontaktfläche 24 längs des Meridians größeren Krümmungsradius. Die um 90 Grad gedrehte Schnittdarstellung der Figur 4 dagegen zeigt den Verlauf der Kontaktfläche 24 längs des Meridians kleineren Krümmungsradius. Die Asphärizität der Kontaktfläche 24 in den beiden Meridianrichtungen kann gleich oder unterschiedlich sein.

Auf ihrer augenabgewandten Seite ist die Applanationslinse 22 in den Figuren 3 und 4 mit einer konvex gekrümmten Form dargestellt. Andere Geometrien der augenabgewandten Linsenseite der Applanationslinse 22 sind ebenso möglich. Beispielsweise kann bei einer abgewandelten Ausführungsform die augenabgewandte Linsenseite der Applanationslinse 22 eine Planfläche sein. Beschränkungen hinsichtlich der Geometrie der augenabgewandten Linsenfläche sind im Rahmen der Erfindung keinesfalls beabsichtigt.

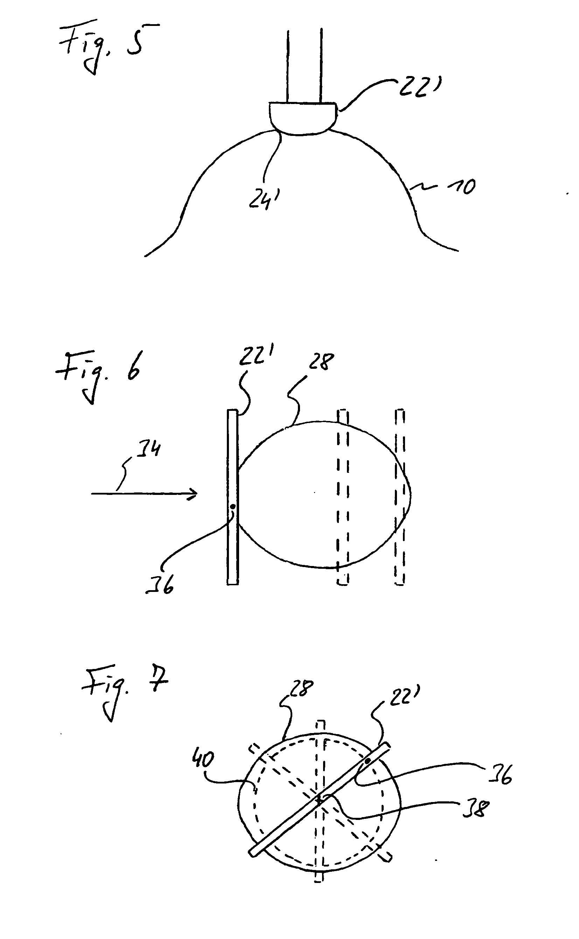

Die Stablinse 22' ist in einer Schnittansicht quer zu ihrer Stablängsrichtung in Figur 5 vergrößert gezeigt. Ihre augenzugewandte Kontaktfläche - zur Unterscheidung von der bitorischen Kontaktfläche 24 der Applanationslinse 22 nunmehr mit 24' bezeichnet - ist asphärisch konvex gekrümmt und kann in Richtung der normal zur Blattebene der Figur 5 verlaufenden Stablängsrichtung der Linse 22 geradlinig oder ebenfalls gekrümmt verlaufen. Bei geradem Längsverlauf der Kontaktfläche 22 ist die Stablinse 22 eine echte Zylinderlinse.

In Figur 6 ist veranschaulicht, wie durch Linearverschiebung der Stablinse 22' quer zu ihrer Stablängsrichtung längs einer Verschieberichtung 34 ein größerer Bereich der Hornhaut 28 überstrichen werden kann. Es versteht sich, dass bei Bewegung der Stablinse 22' auch der in das Auge eingekoppelte Laserstrahl (in Figur 6 durch einen Punkt 36 angedeutet) durch entsprechende Steuerung der oben erwähnten Ablenkmittel mitgeführt werden muss, um nicht an der Stablinse 22' vorbei direkt in die Hornhaut einzufallen. Will man die Hornhaut 28 flächig bearbeiten, kann der Laserstrahl 36 entlang der Längserstreckung der Stablinse 22, also quer zur Vorschubrichtung 34, mittels der Ablenkmittel hin- und herbewegt werden, so dass der Laserstrahl 36 während der Vorschubbewegung der Stablinse 22' fortwährend längs derselben hin- und herwandert. Auf diese Weise kann beispielsweise der für die Flap-Präparation benötigte flächige Tiefenschnitt in der Hornhaut erzeugt werden.

Figur 7 veranschaulicht dagegen die Drehverstellung der Stablinse 22'. Hier wird sie um eine im wesentlichen mittig liegende Drehachse 38 über die Hornhaut 28 gedreht. Wird dabei der Laserstrahl 36 im wesentlichen an derselben drehachsversetzten Längsstelle der Stablinse 22' gehalten, kann ein gestrichelt angedeuteter Zirkularschnitt 40 erzeugt werden.

## Patentansprüche

1. Lasereinrichtung für die ophthalmologische Chirurgie, mit einer gepulsten Laserstrahlung bereitstellenden Laserstrahlungsquelle (14) und Mitteln (18) zur Einkopplung der Laserstrahlung In einen okularen Behandlungsort, wobei die Einkopplungsmittel eine auf eine Augenoberfläche aufzusetzende Applanationslinse (22) umfassen,
**dadurch gekennzeichnet, dass** die Applanationslinse (22) auf ihrer augenzugewandten Seite eine Kontaktfläche (24) besitzt, welche in zwei quer zueinander verlaufenden Hauptmeridianrichtungen jeweils asphärische Kontur mit unterschiedlichen Krümmungsradien hat.

2. Applanationslinse (22) zur Verwendung in einer Lasereinrichtung nach Anspruch 1, **dadurch** gekenntzeichnet, dass sie auf einer zur Anlage an einer Augenoberfläche bestimmten Linsenseite eine Kontaktfläche-(24) besitzt, welche-in zwei quer zueinander verlaufenden Hauptmeridianrichtungen jeweils asphärische kontur mit unterschiedlichen Krümmungsradien hat.

## Claims

1. Laser device for ophthalmological surgery, comprising a laser radiation source (14) providing pulsed laser radiation, and means (18) for coupling the laser radiation into an ocular treatment location, wherein the coupling means include an applanation lens (22) to be placed onto the surface of an eye,
**characterized in that** the applanation lens (22) has a contact surface (24) on its side facing the eye, which contact surface has an aspherical contour with different radii of curvature in the directions of two mutually transverse main meridians.

2. Applanation lens (22) for use in a laser device according to claim 1, **characterized in that** it has a contact surface (24) on a lens side destined for contact with the surface of an eye, which contact surface has an aspherical contour with different radii of curvature in the directions of two mutually transverse main meridians.

## Revendications

1. Dispositif laser pour la chirurgie ophtalmique, comprenant une source de rayonnement laser (14) qui fournit un rayonnement laser pulsé, et des moyens (18) permettant le couplage du rayonnement laser dans un lieu de traitement oculaire, lesdits moyens de couplage comportant une lentille d'aplanation (22) destinée à être posée sur une surface de l'oeil,
**caractérisé en ce que** la lentille d'aplanation (22) possède sur sa face tournée vers l'oeil une surface de contact (24), laquelle présente, dans deux directions de méridiens principaux s'étendant perpendiculairement l'une par rapport à l'autre, un contour asphérique avec des rayons de courbure différents.

2. Lentille d'aplanation (22) destinée à être utilisée dans un dispositif laser selon la revendication 1, **caractérisée en ce qu'**elle possède sur une face de lentille destinée à venir en application sur une surface de l'oeil une surface de contact (24), laquelle présente, dans deux directions de méridiens principaux s'étendant perpendiculairement l'une par rapport à l'autre, un contour asphérique avec des rayons de courbure différents.
